# EUROPÄISCHE PATENTSCHRIFT

(11) **EP 2 809 641 B1**
(45) Veröffentlichungstag und Bekanntmachung des Hinweises auf die Patenterteilung: **20.07.2016**
(21) Anmeldenummer: 13700461.0
(22) Anmeldetag: 15.01.2013
(51) Int. Cl.: C07C 41/01, C07C 41/34, C07C 43/04, C07C 1/20

(54) **VERFAHREN ZUR DIREKTEN DIMETHYLETHERSYNTHESE AUS SYNTHESEGAS**
METHOD FOR DIRECTLY SYNTHESIZING DIMETHYL ETHER FROM SYNTHESIS GAS
PROCÉDÉ DE SYNTHÈSE DIRECTE DE DIMÉTHYLÉTHER À PARTIR DE GAZ DE SYNTHÈSE

(30) Priorität: 31.01.2012 DE 102012001804
(43) Veröffentlichungstag der Anmeldung: 10.12.2014
(73) Patentinhaber: Linde AG, 80331 München (DE)
(72) Erfinder: SCHÖDEL, Nicole, 81477 München (DE); HAIDEGGER, Ernst, 85521 Riemerling (DE); SCHMIGALLE, Holger, 82538 Geretsried (DE); THALLER, Christian, 80687 München (DE); SCHMADERER, Harald, 82515 Wolfratshausen (DE); TOTA, Akos, 81479 München (DE)
(86) Internationale Anmeldenummer: PCT/EP2013/000103
(87) Internationale Veröffentlichungsnummer: WO 2013/113469

(56) Entgegenhaltungen:
- US-B1- 6 458 856
- CHRISTOPHER J ORME ET AL: "Separation of dimethyl ether from syn-gas components by poly(dimethylsiloxane) and poly(4-methyl-1-pentene) membranes", CHEMICAL ENGINEERING JOURNAL, ELSEVIER SEQUOIA, LAUSANNE, CH, Bd. 170, Nr. 1, 10. März 2011 (2011-03-10) , Seiten 178-183, XP028208619, ISSN: 1385-8947, DOI: 10.1016/J.CEJ.2011.03.051 [gefunden am 2011-03-21]

## Beschreibung

Die Erfindung betrifft ein Verfahren zur Herstellung von Dimethylether aus Synthesegas.

Ein solches Verfahren umfasst einen Syntheseschritt, wobei Synthesegas umfassend Wasserstoff und Kohlenmonoxid zu Dimethylether und Kohlendioxid umgesetzt wird, und einen Trennungsschritt, wobei nicht umgesetztes Synthesegas von Kohlendioxid getrennt wird.

Diesbezüglich wird in CHRISTOPHER J ORME ET AL: "Separation of dimethyl ether from syngas components by poly(dimethylsiloxane) and poly(4-methyl-1-pentene) membranes", CHEMICAL ENGINEERING JOURNAL, ELSEVIER SEQUOIA, LAUSANNE, CH, Bd. 170 Nr. 1, 10. März 2011, Seiten 178-183, XP028208619, ISSN: 1385-8947, DOI: 10.1016/J.CEJ.2011.03.051, die Herstellung von Dimethylether und Kohlendioxid ausgehend von Synthesegas offenbart. Weiterhin offenbart auch die US6,458,856B1 die Herstellung von Dimethylether und Kohlendioxid ausgehend von Synthesegas, wobei hier eine absorptive Abtrennung von Synthesegas von CO₂ und DME in einer Kolonne erfolgt.

In einer direkten Dimethylether (DME) Synthese wird in der Hauptreaktion ein H₂/CO Gemisch in einem stöchiometrisch bevorzugten Verhältnis von H₂ und CO von 1:1 an einem bifunktionalen Katalysator zu DME und CO₂ umgesetzt. Neben der Hauptreaktion wird mit geringer Selektivität auch Methanol und Wasser gebildet. Da es sich bei den Reaktionen um Gleichgewichtsreaktionen handelt, kommt es zu keiner vollständigen Umsetzung des H₂/CO Gemisches. Der Reaktoraustrittsstrom setzt sich somit im Wesentlichen aus den Komponenten DME, CO₂, CO, H₂, H₂O und Methanol zusammen. Um einen möglichst hohen Wirtschaftlichkeitsgrad zu gewährleisten, ist es wünschenswert, die Gaskomponenten CO, H₂ und CO₂ zu trennen und stofflich weiter nutzen zu können.

Die Abtrennung von CO₂ von nicht umgesetztem Synthesegas stellt die wesentliche Problemstellung im Trennteil einer einstufigen DME-Anlage dar. Bisherige Lösungsansätze sind mit Nachteilen behaftet.

Die DME Synthesereaktion kann mit einem Wasserstoffüberschuss betrieben werden, um die Bildung von Kohlendioxid zu unterdrücken. Wasserstoff, CO und CO₂ werden nach der Synthesereaktion von den anderen Produkten DME, H₂O und Methanol abgetrennt und der Synthesereaktion wieder zugeführt. Dieser Betrieb der DME Reaktion bei Wasserstoffüberschuss führt jedoch zu einem deutlichen Verlust der Produktivität.

Alternativ kann die DME Synthese nahe am stöchiometrischen Optimum betrieben werden, erfordert aber dann eine Rückführung des Kohlendioxids in die Synthesegaserzeugung (methan- oder erdgasbasierte Synthesegaserzeugung) und eine Rückführung von Kohlenmonoxid und Wasserstoff in die DME-Synthesereaktion. Der hohe apparative und betriebstechnische Aufwand einer separaten CO₂ Abtrennung aus dem H₂/CO Gemisch kann die Vorteile der direkten DME Synthese gegenüber einer indirekten DME Synthese wieder zunichtemachen.

Eine absorptive Abtrennung von CO₂ beispielsweise hat stets mit den folgenden beiden Problemen zu kämpfen. Einerseits muss das Absorptionsmittel eine gute CO₂ Löslichkeit bei moderaten Bedingungen aufweisen und andererseits soll es einen möglichst geringen Dampfdruck besitzen, um mit geringem Aufwand regeneriert werden zu können. Meist wird Methanol in kommerziellen Prozessen zur Entfernung von CO₂ aus Gasströmen verwendet, jedoch wird das Verfahren bei ca. -30°C betrieben, was erhebliche Kosten durch die Kältebereitstellung verursacht. Die Löslichkeit des CO₂ in DME ist deutlich höher als in Methanol. Jedoch weist DME bei moderaten Bedingungen einen hohen Dampfdruck auf. Dies führt dazu, dass ein gewisser DME Anteil mit dem Synthesegas in der DME Synthesereaktion recycelt wird und sich damit die Produktivität verschlechtert. Bei der weiteren Prozesssierung führt der hohe Dampfdruck dazu, dass ein Kompromiss zwischen erhöhtem Druck bzw. niedriger Kopfkondensatortemperatur getroffen werden muss, um das CO₂-DME Gemisch destillativ zu trennen.

In der US 6,458,856 wird die Verwendung eines DME/Methanol Gemisches als Waschmittel beschrieben. Jedoch bestehen die beschriebenen Probleme auch bei der Verwendung dieses Waschmittelgemisches fort.

Hiervon ausgehend liegt daher der vorliegenden Erfindung die Aufgabe zugrunde, ein apparativ einfaches und ökonomisch tragfähiges Verfahren zur Synthese von Dimethylether aus Synthesegas zur Verfügung zu stellen.

Dieses Problem wird durch ein Verfahren mit den Merkmalen des Anspruchs 1 gelöst.

Danach ist vorgesehen, dass das nicht umgesetzte Synthesegas von Kohlendioxid und Dimethylether durch eine Membran getrennt wird, wobei ein vorwiegend wasserstoff-und kohlenmonoxidhaltiges Retentat und ein vorwiegend dimethylether- und kohlendioxidhaltiges Permeat entstehen, und wobei das vorwiegend wasserstoff- und kohlenmonoxidhaltige Retentat dem Syntheseschritt zugeführt wird.

Eine Membran im Sinne der Erfindung ist eine Trennschicht, die zwei (ggf. homogene) Phasen mit unterschiedlicher Zusammensetzung voneinander trennt. Die Membran kann halbdurchlässig bzw. selektiv durchlässig für bestimmte Stoffe sein. Die Membran kann aus Glas oder Kunstoffen wie Keramik oder einem oder mehreren Polymeren bestehen. Für die Verwendung geeignete Polymere sind zum Beispiel Multiblockcopolymere aus Polyethylenglycol und Polyamid oder Polymere auf Silikonbasis wie zum Beispiel Polydimethylsiloxan. Die Membran kann als Flachmembran in Form einer Folie oder als Hohl(faser)membran in Form eines Schlauchs bzw. Rohres ausgebildet sein.

Gemäß einer Ausführungsform der Erfindung ist die Membran durch einen hohen Trennfaktor für Kohlendioxid oder Synthesegas gekennzeichnet. Der Trennfaktor im Sinne der Erfindung bedeutet den Quotienten aus der Konzentration eines Stoffes im Permeat und der in die Membrantrennung eingesetzten Stoffkonzentration. Ein bevorzugter Trennfaktor für Kohlendioxid oder Synthesegas beträgt 0,7, bevorzugter 0,8 oder 0,9.

Eine besonders bevorzugte Membran weist unterschiedliche Trennfaktoren für Kohlendioxid und Synthesegas auf. Diese Membran ist unterschiedlich durchlässig für Kohlendioxid und Synthesegas und/oder weist unterschiedliche Permeationsgeschwindigkeiten für Kohlendioxid und Synthesegas auf. Eine solche Membran ist dazu ausgebildet, Kohlendioxid im Permeat und Synthesegas im Retentat anzureichern oder Kohlendioxid im Retentat und Synthesegas im Permeat anzureichern. Anreichern im Sinne der Erfindung bedeutet insbesondere, dass durch die Membrantrennung mindestens 60%, 70% 80% oder 90% der eingesetzten Stoffe im Permeat oder Retentat vorliegen.

Gemäß einer bevorzugten Ausführungsform der Erfindung ist die Membran durch einen hohen Gasfluss für Kohlendioxid, insbesondere durch eine hohe Permeabilität für Kohlendioxid gekennzeichnet. Ein hoher Gasfluss im Sinne der Erfindung bedeutet eine hohe Permeationsgeschwindigkeit für Kohlendioxid.

Gemäß einer weiteren bevorzugten Ausführungsform der Erfindung ist die Membran durch einen hohen Gasfluss für Dimethylether, insbesondere durch eine hohe Permeabilität für Dimethylether gekennzeichnet. Ein hoher Gasfluss im Sinne der Erfindung bedeutet eine hohe Permeationsgeschwindigkeit für Dimethylether.

Gemäß einer weiteren bevorzugten Ausführungsform besteht die Membran aus mindestens einem Polymer, insbesondere aus Polydimethylsiloxan. Ein Polymer im Sinne der Erfindung kann insbesondere ein Homopolymer oder ein Copolymer sein. Die Membran kann auch aus mehreren verschiedenen Polymeren, sogenannten Polymerlegierungen bestehen.

Wie bereits dargelegt entstehen im Trennungsschritt von Kohlendioxid und nicht umgesetztem Synthesegas ein vorwiegend wasserstoff- und kohlenmonoxidhaltiges Retentat und ein vorwiegend dimethylether- und kohlendioxidhaltiges Permeat.

Ein vorwiegend wasserstoff- und kohlenmonoxidhaltiges Retentat im Sinne der Erfindung bedeutet insbesondere, dass das Retentat zu mindestens 60%, 70% oder 80% (Volumenprozent) aus Wasserstoff und Kohlenmonoxid besteht. Ebenso bedeutet ein vorwiegend dimethylether- und kohlendioxidhaltiges Permeat insbesondere, dass das Permeat zu mindestens 60%, 70% oder 80% (Volumenprozent) aus Kohlendioxid und Dimethylether besteht.

Weiterhin wird, wie bereits dargelegt, das vorwiegend wasserstoff- und kohlenmonoxidhaltige Retentat dem Syntheseschritt zugeführt. Eine Rückführung des Synthesegases zum Syntheseschritt führt zu einer Erhöhung der Ausbeute der Reaktion und einer Reduzierung der anfallenden Abfallproduktmenge.

Gemäß einer weiteren Ausführungsform wird aus dem vorwiegend dimethylether- und kohlendioxidhaltigem Permeat Kohlendioxid abgetrennt. Kohlendioxid kann durch konventionelle Trennverfahren wie etwa Destillation aus dem Permeat entfernt werden. Alternativ kann Kohlendioxid auch durch Amin- oder Alkalicarbonatwäschen, Wäschen mit organischen Lösungsmitteln wie Methanol, N-Methyl-2-pyrrolidon oder Polyethylenglykoldimethylether oder unter Verwendung einer Membran aus dem Permeat abgetrennt werden.

Gemäß einer weiteren Ausführungsform wird das aus dem Permeat abgetrennte Kohlendioxid zur Herstellung von Synthesegas verwendet, wobei Kohlendioxid und Methan zu Wasserstoff und Kohlenmonoxid umgesetzt werden.

Vorteilhafterweise wird vor dem Trennungsschritt, wobei nicht umgesetztes Synthesegas von Kohlendioxid getrennt wird, eine optionale Trennung der flüssigen und der gasförmigen Phase durchgeführt.

Weiterhin kann Dimethlyether in einem Olefinsyntheseschritt zu einem Produkt umfassend Olefine, insbesondere Ethylen und/oder Propylen, umgesetzt werden, wobei Dimethylether bzw. das besagte Permeat direkt dem Olefinsyntheseschritt zugeführt wird, wobei zuvor lediglich CO₂ aus dem Permeat abgetrennt werden kann und sodann das Permeat dem Olefinsyntheseschritt zugeführt werden kann.

Weitere Einzelheiten und Vorteile der Erfindung sollen durch die nachfolgenden Figurenbeschreibungen von Ausführungsbeispielen anhand der Figuren erläutert werden.

Es zeigt:

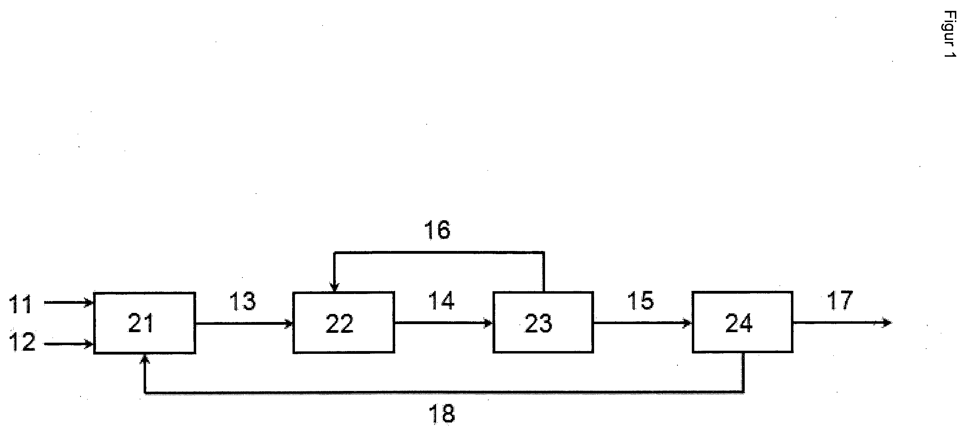

- Fig. 1: ein Schema des erfindungsgemäßen Verfahrens.

### Beispiel 1:

Die vorliegende Erfindung befasst sich insbesondere mit dem Trennteil eines Verfahrens zur direkten DME Synthese (Fig. 1).

In der direkten Dimethylethersynthese 22 wird Synthesegas 13 zu Dimethylether und Kohlendioxid umgesetzt. Der Syntheseproduktgasstrom 14 enthält neben den Produkten auch nicht umgesetztes Synthesegas. Dieses Synthesegas wird mit Hilfe einer Polymermembran vom Dimethylether und Kohlendioxid getrennt 23, wobei das synthesegashaltige Retentat 16 zurück in die DME-Synthese 22 geführt wird. Aus dem verbliebenen dimethylether- und kohlendioxidhaltige Permeat 15 wird anschließend Kohlendioxid 18 abgetrennt und für die Synthesegasherstellung 21 verwendet, wobei Methan 11 und Kohlendioxid 12, 18 zu Synthesegas 13 umgesetzt wird.

Die Auftrennung 23 von Gasströmen mit Hilfe von Polymermembranen ist eine Alternative zu den klassischen Trennverfahren, wie Destillation, Adsorption oder Absorption. Besonders wichtig für die Wirtschaftlichkeit dieses Trennverfahrens ist die Anwendung einer Membran mit einem hohen Trennfaktor und einem hohen Gasfluss. Hochpermeable Polymermembranen, wie PDMS Membranen (Polydimethylsiloxan) eigenen sich gut, um das Stoffgemisch 14 aus dem DME-Reaktor 22 (H₂/CO/CO₂/DME/Methanol) in die folgenden beiden Fraktionen aufzuspalten:
- Retentat 16: H₂, CO, +Spuren DME und CO₂
- Permeat 15: DME, CO₂, Methanol + Spuren H₂ und CO.

Die Trennaufgabe wird dadurch wesentlich erleichtert. Die Retentat-Fraktion 16 kann so direkt dem Reaktionsteil 22 zugeführt werden. Die Permeat-Fraktion 15 wird mittels konventioneller Trennverfahren destillativ aufgetrennt 24 und CO₂ 18 wird in den Synthesegasteil 21 zurückgeführt. Der Aufwand der Aufreinigung des DME 17 ist stark abhängig von der Anwendung (Treibmittel, Lösungsmittel, LPG-Beimischung, Treibstoff, Einsatzstoff für Olefinsynthese).

**Bezugszeichenliste:**

| | |
|---|---|
| 11 | Methan |
| 12 | CO₂ |
| 13 | Synthesegas (H₂, CO) |
| 14 | Syntheseproduktgasstrom (DME, CO₂, H₂, CO, MeOH) |
| 15 | Permeat (DME, CO₂) |
| 16 | Retentat (H₂, CO) |
| 17 | DME |
| 18 | CO₂ |
| 21 | Synthesegasherstellung |
| 22 | Direkte DME-Synthese |
| 23 | Trennung CO₂ und Synthesegas |
| 24 | Trennung CO₂ und DME |

## Patentansprüche

1. Verfahren zur Herstellung von Dimethylether (17) aus Synthesegas (13), umfassend
- einen Syntheseschritt (22), wobei Synthesegas (13) umfassend Wasserstoff und Kohlenmonoxid zu Dimethylether und Kohlendioxid umgesetzt wird, und
- einen Trennungsschritt (23), wobei nicht umgesetztes Synthesegas von Kohlendioxid und Dimethylether getrennt wird, wobei nicht umgesetztes Synthesegas von Kohlendioxid und Dimethylether durch eine Membran getrennt wird, wobei ein vorwiegend wasserstoff- und kohlenmonoxidhaltiges Retentat (16) und ein vorwiegend dimethylether-und kohlendioxidhaltiges Permeat (15) entstehen,
- und wobei das vorwiegend wasserstoff- und kohlenmonoxidhaltige Retentat (16) dem Syntheseschritt (22) zugeführt wird.

2. Verfahren nach Anspruch 1, **dadurch gekennzeichnet, dass** die Membran einen Trennfaktor für Kohlendioxid oder Synthesegas aufweist, der zumindest 0,7 beträgt, bevorzugt zumindest 0,8, bevorzugt zumindest 0,9.

3. Verfahren nach Anspruch 1 oder 2, **dadurch gekennzeichnet, dass** die Membran einen hohen Gasfluss für Kohlendioxid, insbesondere eine hohe Permeabilität für Kohlendioxid aufweist.

4. Verfahren nach Anspruch 3, **dadurch gekennzeichnet, dass** die Membran einen hohen Gasfluss für Dimethylether, insbesondere eine hohe Permeabilität für Dimethylether aufweist.

5. Verfahren nach einem der Ansprüche 1 bis 4, **dadurch gekennzeichnet, dass** die Membran zumindest ein Polymer aufweist, insbesondere Polydimethylsiloxan.

6. Verfahren nach einem der vorhergehenden Ansprüche, **dadurch gekennzeichnet, dass** aus dem vorwiegend dimethylether- und kohlendioxidhaltigen Permeat (15) Kohlendioxid (18) abgetrennt wird.

7. Verfahren nach Anspruch 6, **dadurch gekennzeichnet, dass** aus dem Permeat (15) abgetrenntes Kohlendioxid (18) zur Herstellung (21) von Synthesegas verwendet wird.

8. Verfahren nach einem der vorangegangenen Ansprüche, **dadurch gekennzeichnet, dass** vor dem Trennungsschritt (23) eine Trennung der flüssigen und der gasförmigen Phase durchgeführt wird.

9. Verfahren nach einem der vorherigen Ansprüche, **dadurch gekennzeichnet, dass** Dimethylether (17) in einem Olefinsyntheseschritt zu einem Produkt umfassend Olefine, insbesondere Ethylen und/oder Propylen, umgesetzt wird, wobei Dimethylether (17) oder das Permeat (15) direkt dem Olefinsyntheseschritt zugeführt wird, oder wobei lediglich CO₂ aus dem Permeat (15) abgetrennt wird und sodann das Permeat (15) dem Olefinsyntheseschritt zugeführt wird.

## Claims

1. Process for preparation of dimethyl ether (17) from synthesis gas (13), comprising
- a synthesis step (22), wherein synthesis gas (13) comprising hydrogen and carbon monoxide is converted into dimethyl ether and carbon dioxide, and
- a separation step (23), wherein unconverted synthesis gas is separated from carbon dioxide and dimethyl ether, wherein unconverted synthesis gas is separated from carbon dioxide and dimethyl ether by a membrane, wherein a predominantly hydrogen- and carbon monoxide-containing retentate (16) and a predominantly dimethyl ether- and carbon dioxide-containing permeate (15) are produced,
- and wherein the predominantly hydrogen- and carbon monoxide-containing retentate (16) is fed into the synthesis step (22).

2. Process according to Claim 1, **characterized in that** the membrane has a separation factor for carbon dioxide or synthesis gas of at least 0.7, preferably at least 0.8, preferably at least 0.9.

3. Process according to Claim 1 or 2, **characterized in that** the membrane has a high gas flux for carbon dioxide, especially a high permeability for carbon dioxide.

4. Process according to Claim 3, **characterized in that** the membrane has a high gas flux for dimethyl ether, especially a high permeability for dimethyl ether.

5. Process according to any of Claims 1 to 4, **characterized in that** the membrane comprises at least a polymer, especially polydimethylsiloxane.

6. Process according to any preceding claim, **characterized in that** carbon dioxide (18) is separated from the predominantly dimethyl ether- and carbon dioxide-containing permeate (15).

7. Process according to Claim 6, **characterized in that** carbon dioxide (18) separated from the permeate (15) is used for production (21) of synthesis gas.

8. Process according to any preceding claim, **characterized in that** the liquid and gaseous phases are separated before the separation step (23).

9. Process according to any preceding claim, **characterized in that** dimethyl ether (17) is converted in an olefin synthesis step into a product comprising olefins, especially ethylene and/or propylene, wherein dimethyl ether (17) or the permeate (15) is fed directly to the olefin synthesis step, or wherein merely CO₂ is separated from the permeate (15) and then the permeate (15) is fed to the olefin synthesis step.

## Revendications

1. Procédé de fabrication d'éther diméthylique (17) à partir d'un gaz de synthèse (13), comprenant :
- une étape de synthèse (22), lors de laquelle un gaz de synthèse (13) comprenant de l'hydrogène et du monoxyde de carbone est transformé en éther diméthylique et dioxyde de carbone, et
- une étape de séparation (23), lors de laquelle le gaz de synthèse non réagi est séparé du dioxyde de carbone et de l'éther diméthylique, le gaz de synthèse non réagi étant séparé du dioxyde de carbone et de l'éther diméthylique par une membrane, un rétentat (16) contenant principalement de l'hydrogène et du monoxyde de carbone et un perméat (15) contenant principalement de l'éther diméthylique et du dioxyde de carbone se formant,
- et le rétentat (16) contenant principalement de l'hydrogène et du monoxyde de carbone étant introduit dans l'étape de synthèse (22).

2. Procédé selon la revendication 1, **caractérisé en ce que** la membrane présente un facteur de séparation pour le dioxyde de carbone ou le gaz de synthèse qui est d'au moins 0,7, de préférence d'au moins 0,8, de préférence d'au moins 0,9.

3. Procédé selon la revendication 1 ou 2, **caractérisé en ce que** la membrane présente un flux de gaz élevé pour le dioxyde de carbone, notamment une perméabilité élevée pour le dioxyde de carbone.

4. Procédé selon la revendication 3, **caractérisé en ce que** la membrane présente un flux de gaz élevé pour l'éther diméthylique, notamment une perméabilité élevée pour l'éther diméthylique.

5. Procédé selon l'une quelconque des revendications 1 à 4, **caractérisé en ce que** la membrane comprend au moins un polymère, notamment le polydiméthylsiloxane.

6. Procédé selon l'une quelconque des revendications précédentes, **caractérisé en ce que** le dioxyde de carbone (18) est séparé du perméat (15) contenant principalement de l'éther diméthylique et du dioxyde de carbone.

7. Procédé selon la revendication 6, **caractérisé en ce que** le dioxyde de carbone (18) séparé du perméat (15) est utilisé pour la fabrication (21) du gaz de synthèse.

8. Procédé selon l'une quelconque des revendications précédentes, **caractérisé en ce qu'**une séparation de la phase liquide et de la phase gazeuse est réalisée avant l'étape de séparation (23).

9. Procédé selon l'une quelconque des revendications précédentes, **caractérisé en ce que** l'éther diméthylique (17) est mis en réaction dans une étape de synthèse d'oléfines pour former un produit comprenant des oléfines, notamment de l'éthylène et/ou du propylène, l'éther diméthylique (17) ou le perméat (15) étant introduit directement dans l'étape de synthèse d'oléfines ou seul le CO₂ étant séparé du perméat (15), puis le perméat (15) étant introduit dans l'étape de synthèse d'oléfines.
